# EUROPÄISCHE PATENTSCHRIFT

(11) **EP 2 455 893 B1**
(45) Veröffentlichungstag und Bekanntmachung des Hinweises auf die Patenterteilung: **08.07.2015**
(21) Anmeldenummer: 11450143.0
(22) Anmeldetag: 16.11.2011
(51) Int. Cl.: G06K 17/00, A61B 19/00

(54) **Anordnung und Verfahren zum Auslesen und Verarbeiten multi-parametrischer Daten aus unterschiedlichen Quellen**
Design and method for reading and processing multi-parameter data from various sources
Agencement et procédé de lecture et de traitement de données multi-paramétriques provenant de sources différentes

(30) Priorität: 17.11.2010 AT 18962010
(43) Veröffentlichungstag der Anmeldung: 23.05.2012
(73) Patentinhaber: AIT Austrian Institute of Technology GmbH, 1220 Wien (AT)
(72) Erfinder: Hayn, Dieter, 8010 Graz (AT); Modre-Osprian, Robert, 8563 Ligist (AT); Schreier, Günther, 8010 Graz (AT)
(74) Vertreter: Wildhack & Jellinek

(56) Entgegenhaltungen:
- EP-A2- 2 218 421

## Beschreibung

Die Erfindung bezieht sich auf ein Verfahren und eine Vorrichtung zur Erfassung und Verarbeitung von Daten.

Die Erfassung und Verarbeitung von Daten aus unterschiedlichen Quellen und ihre Verknüpfung miteinander ist ein häufiges Problem in vielen Anwendungsszenarien. Datenquellen können in diesem Zusammenhang Gegenstände mit einem statischen Inhalt sein (z.B. ein Personalausweis, das Etikett mit der Seriennummer eines Gerätes, ein Herz-As aus einem Kartenspiel) oder solche mit dynamischen, veränderlichen Daten (z.B. ein Thermometer oder eine Uhr).

Eine Untergruppe von zu sammelnden Daten sind medizinische Daten, wie sie täglich in Krankenhäusern, Arztpraxen, Pflegeeinrichtungen und auch bei Patienten zu Hause erfasst werden. Beispielsweise werden beim Homemonitoring von chronischen Krankheiten medizinische Messwerte (Blutdruck, Körpergewicht, Blutzucker, etc.), subjektives Wohlbefinden, Einnahme von Medikamenten etc. erfasst. Die meist älteren Patienten müssen und sollen dabei aktiv ins Monitoring eingebunden werden, indem sie selbst die benötigten Daten messen und mit technischen Hilfsmitteln ihrem Arzt zur Verfügung stellen.

Der (meist ältere) Patient soll im Zuge von Homemontoring üblicherweise Daten erfassen. Mit diesen Daten können dann unterschiedliche Verarbeitungsschritte durchgeführt werden, wie z.B: Übertragung an den Arzt, Übertragung an den Arzt mit Bitte um Rückruf, Übertragung an den Arzt und Auslösen eines Alarms (Notfall), Übertragung an Verwandte, Empfehlen einer weiteren Vorgehensweise (z.B. Dosierung eines Medikamentes), Darstellung von Ergebnissen (z.B. Gewichtsverlauf der letzten 4 Wochen), etc.

Problematisch dabei ist jedoch, dass sowohl das Sammeln der Daten als auch das Auslösen der Anschließenden Verarbeitungsschritte für den Patienten oft kompliziert, wenig intuitiv ist, und die eingesetzten Technologien nicht für ältere Personen optimiert sind.

Um Daten mit technischen Hilfsmitteln zu erfassen und zu verarbeiten, müssen Sie auf irgendeine Art und Weise ausgelesen werden. Eine Möglichkeit wäre das manuelle Eingeben der Daten in das technische Gerät. Heute gibt es aber auch zahlreiche Technologien, mit denen statische Daten (RFID, Barcode-Scanner, Kartenlesegerät, etc.) oder dynamische Daten (Bluetooth, WIFI, USB) von einer Quelle an ein Zielgerät übertragen werden können. Stand der Technik sind zahlreiche Geräte, die über unterschiedliche Schnittstellen Daten erfassen und verarbeiten. Dazu gehören Computer oder Mobiltelefone, die Daten von unterschiedlichsten Medien empfangen und verarbeiten können.

Die Übertragung der Daten an diese Geräte ist jedoch an wenig intuitive Verfahren gekoppelt, wie z.B. das Anstecken eines Kabels und die manuelle Auswahl der gewünschten Daten mit Hilfe eines GUls, oder das Herstellen einer Bluetooth-Verbindung und die Übertragung der Daten von einem Gerät ans andere etc.

Stand der Technik sind auch Behälter, die jederzeit ihren Inhalt auslesen können. So gibt es Kühltruhen mit RFID-Technik, welche Gegenstände, die zuvor mit RFID-Tags markiert wurden, erkennen können. Weiter is EP 2 218 421 A2 als Stand der Technik bekannt.

Derartige Behälter haben allerdings nicht den Zweck, Daten zu verarbeiten und sie können nur einen einzigen Typ von Daten auslesen (Identifikationscodes mittels RFID). Nach einem ähnlichen Prinzip arbeiten auch RFID-basierte Zutrittskontroll-Systeme, z.B. für Stadien, nur dass hier das Auslesen nicht passiert, wenn die Datenträger (also Eintrittskarten) bereits im Behälter (also Stadion) sind, sondern bereits zu jenem Zeitpunkt, wo sie das Stadion betreten.

Weiters sind Vorrichtungen Stand der Technik, die Gegenstände in einem Behälter auslesen können (z.B. in einem Einkaufswagen) und auf Grund der identifizierten Gegenstände eine Datenverarbeitung ausführen können (z.B. den Preis aller Gegenstände addieren und die identifizierten Gegenstände aus der Inventarliste löschen). Dabei kann die Ausleseeinheit außerhalb sein, oder sie ist selbst Teil des Behälters (des Einkaufswagens). Die Art der Verarbeitung kann bei derartigen Behältern allerdings nicht durch den Benutzer beeinflusst werden.

Im Homemonitoring chronischer Daten sind Technologien bekannt, in denen ein Terminal (z.B. Mobiltelefon) Daten von anderen Geräten holt (z.B. via Bluetooth, RFID, lrDA, etc.) und anschließend verarbeitet. Das Holen der Daten erfolgt dabei entweder automatisch ohne Einflussnahme des Patienten (z.B. bei Bluetooth) oder durch manuelles Auslesen von statischen oder dynamischen Datenträgern, indem der Patient die Datenquelle mit dem Mobiltelefon berührt (RFID).

Je mehr unterschiedliche Daten gesammelt werden sollen und je mehr unterschiedliche Technologien dabei verwendet werden, umso komplizierter wird auch die Datensammlung, umso schwieriger wird es zu wissen, welche Daten bereits ausgelesen wurden und welche noch nicht und umso mehr Fehlerquellen können sich während des Datensammelns auftun.

Der Erfindung liegt daher die Aufgabe zugrunde, Daten auf intuitive Weise zu erfassen und entsprechend den Wünschen des Anwenders zu verarbeiten, sodass auch ältere Personen ohne Probleme die Datenerfassung durchführen können.

Speziell beim Homemonitoring chronischer Krankheiten ergibt sich durch die zunehmende Anzahl an Sensoren bzw. Messgeräten sowie Messwerten das Problem, dass die Datenerfassung, Übermittlung und Zuordnung zu weiteren Prozessen derzeitiger Systeme in Bezug auf Einfachheit,

Gebrauchstauglichkeit, Wartbarkeit, Sicherheit und Wirtschaftlichkeit ungenügend sind.

Daten als solches sind ein schwer greifbares Objekt. Ginge es darum, Gegenstände zu sammeln, würde der Sammler alle benötigten Dinge zusammensuchen und z.B. in einen Behälter legen. Diese Vorgehensweise ist jedem sehr geläufig und daher vollkommen intuitiv. Ziel ist es daher, eine gleich intuitive Methode zur Erfassung und Verarbeitung von Daten zu finden.

Die Aufgabe wird erfindungsgemäß durch ein Verfahren nach Anspruch 1, sowie eine Vorrichtung nach Anspruch 8 gelöst. Gegenstände, die heterogene Daten gespeichert haben, werden in oder auf einen Behälter gelegt, die auf den Gegenständen gespeicherten Daten werden automatisiert ausgelesen und anschließend werden abhängig von zumindest einem der in oder auf den Behälter gelegten Gegenstände - unterschiedliche Verarbeitungsschritte ausgeführt. Der Behälter kann dabei z.B. ein Koffer, eine Lade, eine Matte etc. sein.

Beispielsweise kann neben Messgeräten für Blutdruck, Körpergewicht oder Blutzucker eine RFID-Karte für den Verarbeitungsschritt "Daten an den Arzt senden" als aktionsbestimmender Gegenstand in oder auf den Behälter gelegt werden. Wird als Aktion "Statistik anzeigen" in oder auf den Behälter gelegt, kann auf

einem Anzeigegerät (z.B. am Behälter, auf einem im Behälter befindlichen Gerät oder auf einem externen Gerät, z.B. einem Fernseher oder elektronischen Bilderrahmen) eine statistische Auswertung der im Koffer befindlichen Daten angezeigt werden. Mit Hilfe von Aktionskarten wie "Insulinbedarf anzeigen" könnten Therapieempfehlungen angezeigt werden, etc.

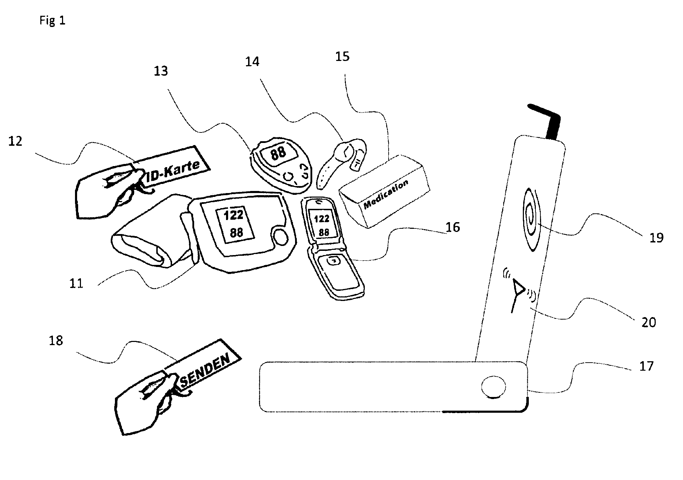

Eine mögliche Ausführungsform, die das erfundene Verfahren anwendet, ist in Fig. 1 dargestellt. Eine Person legt alle für das Monitoring notwendigen Gegenstände wie eine ID-Karte (11), ein Blutdruckmessgerät (12), ein Blutzuckermessgerät (13), eine Uhr (14), Medikamentenschachtel mit RFID-Tag (15), Mobiltelefon (16) in einen Behälter (17). Schließlich kommt noch ein aktionsbestimmender Gegenstand (18), z.B. eine Karte "SENDEN" dazu und beim Schließen des Behälters werden die von diesen Objekten gespeicherten Daten mittels geeigneter RFID-Antenne (19) automatisch ausgelesen und vom Inhalt dieser Daten abhängig über eine Sendeeinheit (20) an eine Datenzentrale zur Weiterverarbeitung geschickt.

Die Verarbeitung kann z.B. starten, wenn der Behälter zugemacht wird, oder wenn ein aktionsbestimmender Gegenstand in den Behälter gelegt wird.

Durch Kombination mehrerer aktionsbestimmender Gegenstände kann das Verhalten des Behälters verändert werden. Z.B. kann durch Hinzufügen der Karte "NOTFALL" zur "SENDEN"-Karte ein Arzt oder die Rettung alarmiert werden, während durch die Karte "Bitte um Rückruf" lediglich Feedback vom Arzt zu den Daten gewünscht werden kann.

Die aktionsbestimmenden Gegenstände müssen nicht unbedingt einzig die Funktion der Aktionsauslösung haben. Es können auch unterschiedliche Aktionen dadurch ausgelöst werden, dass gewisse Aktionen an andere Gegenstände gekoppelt sind. Z.B. kann durch Einlegen je einer Identifikationskarte eines Patienten und eines Arztes dem Arzt Zugriff auf die Daten eines Patienten gegeben werden (Aktion "Zugriff gewähren"). Oder ein Arzt kann Identifikationskarten von mehreren Patienten in den Behälter legen (z.B. aller Mitglieder einer Familie), um einen statistischen Vergleich der Daten all dieser Patienten zu erhalten (z.B. um genetische Zusammenhänge zu erkennen -Aktion "Statistischer Vergleich").

Der Behälter selbst kann auch Sensoren enthalten, die z.B. Umgebungsvariablen bestimmen (Luftdruck, Temperatur, Feuchtigkeit, geographische Position, etc.), die in vielen Fragestellungen von großer Bedeutung sein können. So ist z.B. bekannt, dass Luftdruck und Temperatur großen Einfluss auf das Wohlbefinden haben und daher in einem umfassenden Homemonitoring von chronisch Kranken erfasst werden sollten. Mittels in den Behälter eingebauter Sensoren können aber auch Informationen über den Behälter selbst erfasst werden. Z.B. darüber, ob er liegt oder steht, ob er gerade Richtung Süden zeigt, welches Gewicht er hat, etc. Und die Sensoren können auch Eigenschaften von den Gegenständen, die in ihn gelegt werden, bestimmen, wie z.B. ihr Gewicht (z.B. bei Flüssigkeitsbehältern zur Bestimmung des Füllstandes), ihre Orientierung (z.B. bei einem Würfel, um zu bestimmen, welcher Wert gewürfelt wurde), ihres Ortes innerhalb des Behälters (z.B. wenn der Behälter in mehrere Bereiche mit unterschiedlichen unterteilt ist, z.B. "nur für mich sichtbar", "darf auch mein Arzt sehen", "für jeden sichtbar").

Um unhandliche Gegenstände auszulesen, können anstelle der Gegenstände selbst auch Gegenstände in oder auf den Behälter gelegt werden, die auf die Gegenstände selbst verweisen. Z.B. kann anstelle eines Bluetooth-fähigen Messgerätes eine RFID-Karte mit der Bluetooth-Adresse des Messgerätes in oder auf den Behälter gelegt werden. Beim Auslesen der Daten wird die Bluetooth-Adresse erkannt und im Zuge der Verarbeitung werden über Bluetooth die Daten vom Messgerät ausgelesen, ohne dass das Gerät selbst im Behälter war.

Unter den in oder auf den Behälter gelegten Gegenständen können sich auch Gegenstände befinden, die selbst Datenverarbeitungen durchführen können. Z.B. könnte das ein Mobiltelefon sein, welches dann auch Teile der Verarbeitung (z.B. die Datenübertragung) oder auch die gesamte Verarbeitung durchführen könnte.

Die in oder auf den Behälter gelegten Gegenstände können nicht nur die Aktion selbst auslösen, sondern auch Parameter für die Aktion beinhalten. Z.B. kann ein Gegenstand den URL beinhalten, zu dem Daten geschickt oder von dem Daten abgerufen werden, es kann eine von z.B. drei Karten dazugelegt werden (z.B. rot, gelb, grün) um die Dringlichkeit einer Anfrage anzugeben, es kann der Speicherort, an den Daten gespeichert werden sollen, von einem der Gegenstände entnommen werden. Oder es kann im Fall von Visualisierungen (z.B. Therapie-Empfehlung, Statistik) eine Karte dazugelegt werden, welche die Schriftgröße bei der Darstellung beeinflusst.

## Patentansprüche

1. Verfahren zur Erfassung und Verarbeitung von Daten, wobei die Daten auf unterschiedlichen Gegenständen (11,12,13,14,15,16,18), wie z.B. Messgeräten oder RFID-Karten, gespeichert sind, welche ein Interface zum Auslesen der Daten besitzen, z.B. über NFC, RFID, Bluetooth, WLAN, ZigBee, ANT, IrDA, Zeroconf, WPan, Z-Wave, Bonjour oder EnOcean, wobei als Auslesegerät (19) ein Behälter (17) verwendet wird, z.B. ein Koffer oder eine spezielle Ablagefläche oder eine Sensormatte, in oder auf welchen alle die Daten speichernden Gegenstände gelegt werden und die Daten jener Gegenstände ausgelesen werden, welche in oder auf den Behälter gelegt wurden, **dadurch gekennzeichnet, dass** die Daten auf mindestens einem (18) der in oder auf den Behälter gelegten Gegenstände, z.B. einer RFID-Karte, bestimmen, welche Verarbeitungsschritte durchgeführt werden sollen.

2. Verfahren nach Anspruch 1, **dadurch gekennzeichnet,**
**dass** der Behälter selbst mit integrierten Sensoren auch Daten über sich selbst-z.B. geographische Position, Orientierung, Temperatur, etc. - und/oder über die in oder auf ihn gelegten Gegenstände-z.B. Temperatur, Position innerhalb des Behälters, Orientierung, etc. - und/oder über seine Umgebung - z.B. Temperatur, Luftdruck, Uhrzeit etc. -ermitteln kann und diese Daten die Verarbeitung beeinflussen können.

3. Verfahren nach einem der Ansprüche 1 bis 2, **dadurch gekennzeichnet,**
**dass** das Auslesen und/oder die Verarbeitung gestartet werden, sobald der Behälter geschlossen wurde und/oder sobald mindestens ein vordefinierter Gegenstand und/oder sobald eine vordefinierte Anzahl an Gegenständen in oder auf den Behälter gelegt wurden.

4. Verfahren nach einem der Ansprüche 1 bis 3, **dadurch gekennzeichnet,**
**dass** unter den die Daten speichernden Gegenständen Gegenstände sind, die auf andere Gegenstände, welche nicht in oder auf den Behälter gelegt wurden, verweisen, und dass im Zuge der Verarbeitung auch Daten von jenen Geräten ausgelesen werden, auf welche diese Gegenstände verweisen.

5. Verfahren nach einem der Ansprüche 1 bis 4, **dadurch gekennzeichnet,**
**dass** zumindest einer der in oder auf den Behälter gelegten Gegenstände ein Gerät mit einer Recheneinheit, wie z.B. Mobiltelefon, Smartphone, Netbook, Computer, Laptop, PDA, Uhr und/oder Tablet-PC ist und mindestens einer der in oder auf den Behälter gelegten Gegenstände zumindest Teile der Verarbeitung durchführt.

6. Verfahren nach einem der Ansprüche 1 bis 5, **dadurch gekennzeichnet,**
**dass** im Zuge der Verarbeitung eine Übertragung der Daten an ein anderes Gerät oder an mehrere andere Geräte und/oder eine Visualisierung am Behälter selbst und/oder auf einem anderen Gerät und/oder eine statistische Aufbereitung und/oder eine Speicherung und/oder ein Löschen oder Überschreiben der Daten durchgeführt wird, wobei einzelne Verarbeitungs- Parameter den Daten der in oder auf den Behälter gelegten Gegenständen entnommen werden können, wie z.B. der Speicherort beim Speichern, die Schriftgröße bei der Visualisierung oder das Zielgerät oder die Zieladresse bei der Datenübertragung.

7. Verfahren nach einem der Ansprüche 1 bis 6, **dadurch gekennzeichnet,**
**dass** unter den Daten Identifikationsdaten von Personen sind und dass die Art der Verarbeitung der Daten von der Kombination der mittels der Daten identifizierten Personen abhängt, indem z.B. durch Verwendung einer Patienten-Identifikationskarte und einer Arzt-Identifikationskarte dem Arzt Daten des Patienten geschickt werden oder durch Verwendung mehrerer Patienten-Identifikationskarten ein statistischer Vergleich der Daten aller identifizierten Patienten durchgeführt wird.

8. Vorrichtung zur Erfassung und Verarbeitung von Daten, insbesondere zur Durchführung des Verfahrens nach einem der Ansprüche 1 bis 7, bestehend aus unterschiedlichen Daten speichernden Gegenständen (11, 12, 13, 14, 15, 16, 18), wie z.B. Messgeräten oder RFID-Karten, welche ein Interface zum Auslesen der Daten besitzen, z.B. über NFC, RFID, Bluetooth, WLAN, ZigBee, ANT, IrDA, Zeroconf, WPan, Z-Wave, Bonjour oder EnOcean, und aus einem Behälter (17), z.B. einen Koffer, eine Lade, eine spezielle Ablagefläche oder eine Sensormatte, der eine Ausleseeinheit besitzt, welche ausschließlich Daten von Gegenständen ausliest, die sich im oder auf den Behälter befinden, und wobei die Vorrichtung eine Verarbeitungseinheit enthält,
**dadurch gekennzeichnet, dass** die Daten auf mindestens einem (18) der in oder auf den Behälter gelegten Gegenstände, z.B. einer RFID-Karte, bestimmen, welche Verarbeitungsschritte in der Verarbeitungseinheit durchgeführt werden sollen.

9. Vorrichtung nach Anspruch 8, **dadurch gekennzeichnet,**
**dass** Sensoren in den Behälter integriert sind, welche Daten über den Behälter selbst - z.B. geographische Position, Orientierung, Temperatur, etc. - und/oder über die in oder auf ihn gelegten Gegenstände - z.B. Temperatur, Position innerhalb des Behälters, Orientierung, etc. - und/oder über seine Umgebung-z.B. Temperatur, Luftdruck, Uhrzeit etc. -ermitteln und an die Verarbeitungseinheit übergeben, sodass diese Daten die Verarbeitung beeinflussen können.

10. Vorrichtung nach einem der Ansprüche 8 bis 9, **dadurch gekennzeichnet,**
**dass** das Auslesen und/oder die Verarbeitung gestartet werden, sobald der Behälter geschlossen wurde und/oder sobald mindestens ein vordefinierter Gegenstand und/oder sobald eine vordefinierte Anzahl an Gegenständen in oder auf den Behälter gelegt wurden.

11. Vorrichtung nach einem der Ansprüche 8 bis 10, **dadurch gekennzeichnet,**
**dass** unter den die Daten speichernden Gegenständen Gegenstände sind, die auf andere Gegenstände, welche nicht in oder auf den Behälter gelegt wurden, verweisen, und dass im Zuge der Verarbeitung mit einer zweiten Ausleseeinheit auch Daten von jenen Geräten ausgelesen werden, aufwelche diese Gegenstände verweisen.

12. Vorrichtung nach einem der Ansprüche 8 bis 11, **dadurch gekennzeichnet,**
**dass** zumindest einer der in oder auf den Behälter gelegten Gegenstände ein Gerät mit einer Recheneinheit, wie z.B. Mobiltelefon, Smartphone, Netbook, Computer, Laptop, PDA, Uhr und/oder Tablet-PC ist und mindestens einer der in oder auf den Behälter gelegten Gegenstände zumindest Teile der Verarbeitung durchführt.

13. Vorrichtung nach einem der Ansprüche 8 bis 12, **dadurch gekennzeichnet,**
**dass** die Verarbeitungseinheit eine Übertragungseinheit beinhaltet, welche Daten an ein anderes Gerät oder an mehrere andere Geräte überträgt und/oder eine Visualisierungseinheit, die am Behälter selbst und/oder auf einem anderen Gerät sein kann, und/oder eine statistische Aufbereitungseinheit und/oder eine Speichereinheit und/oder ein Schreibeeinheit, welche die Daten der im oder auf den Behälter befindlichen Gegenstände Ändern oder Löschen kann, wobei einzelne VerarbeitungsParameter den Daten der in oder auf den Behälter gelegten Gegenständen entnommen werden können, wie z.B. der Speicherort beim Speichern, die Schriftgröße bei der Visualisierung oder das Zielgerät oder die Zieladresse bei der Datenübertragung.

14. Vorrichtung nach einem der Ansprüche 8 bis 13 **dadurch gekennzeichnet,**
**dass** unter den Daten Identifikationsdaten von Personen sind und dass die Art der Verarbeitung der Daten von der Kombination der mittels der Daten identifizierten Personen abhängt, indem z.B. durch Verwendung einer Patienten-Identifikationskarte und einer Arzt-Identifikationskarte dem Arzt Daten des Patienten geschickt werden oder durch Verwendung mehrerer Patienten-Identifikationskarten ein statistischer Vergleich der Daten aller identifizierten Patienten durchgeführt wird.

## Claims

1. A method for recording and processing data, said data being stored on different objects (11, 12, 13, 14, 15, 16, 18), such as measuring instruments or RFID cards, which have an interface for reading out the data, for example via NFC, RDID, Bluetooth, WLAN, ZigBee, ANT, IrDA Zeroconf, WPan, Z-Wave, Bonjour or EnOcean, the reading instrument (19) used to being a container (17), such as a suitcase or a special laying area or a sensing area, in or on which all the objects storing data may be laid, and the data of those objects, which have been laid in or on the container, are read, **characterized in that** the data determine, on at least one (18) of the objects laid in or on the container, for example an RFID card, which processing steps have to be carried out.

2. The method according to claim 1, **characterized in that** the container itself may also determine with integrated sensors data about itself - such as the geographic position, the orientation, the temperature, etc. - and/or about the objects laid in or on said container - such as the temperature, the position inside the container, the orientation, etc. - and/or about its environment - such as the temperature, the air pressure, the time, etc. - and these data may influence the processing.

3. The method according to any of claims 1 to 2, characterizing that reading and/or processing begin as soon as the container has been closed and/or as soon as at least one predefined object and/or as soon as a predefined number of objects have been laid in or on the container.

4. The method according to any of claims 1 to 3, **characterized in that**, among the objects storing the data appear objects which refer back to other objects which have not been laid in or on the container, and **in that** during the processing, data of the instruments back to which these objects refer, are also read.

5. The method according to any of claims 1 to 4, characterizing that at least one of the objects laid in or on the container is a device with a computing unit, such as a mobile phone, a smartphone, a portable minicomputer, a computer, a portable computer, a PDA, a watch and/or a tablet, and at least one of the objects laid in or on the container carries out at least portions of the processing.

6. The method according to any of claims 1 to 5, **characterized in that**, during processing, are carried out transmission of the data to another device or to several other devices, and/or viewing on the container itself and/or on another device, and/or statistically preparing and/or backing up and/or suppressing or deleting the data, different processing parameters being able be extracted from the data of the objects laid in or on the container, such as the backup storage location, the size of the characters for viewing or the target device or the target address for transmitting the data.

7. The method according to any of claims 1 to 6, **characterized in that**, among the data, appear data for identifying persons and **in that** the data processing mode depends on the combination of the persons identified by means of the data, for example resulting from the use of an identification card of a patient and of an identification card of a physician, data of the patient being sent to the physician, or from the use of several patient identification cards, statistical comparison of the data of all the identified patients being carried out.

8. A device for recording and processing data, in particular for applying the method according to one of claims 1 to 7, consisting of different objects (11, 12, 13, 14, 15, 16, 18) storing data, such as measuring instruments or RFID cards, which have an interface for reading out data for example via NFC, RDID, Bluetooth, WLAN, ZigBee, ANT, IrDA Zeroconf, WPan, Z-Wave, Bonjour or EnOcean, and of a container (17), such as a suitcase, a box, a special laying area or a sensing mat, which has a reading unit which exclusively reads data of the objects which are laid in or on the container, and said device containing a processing unit, **characterized in that** the data determine, on at least one (18) of the objects laid in or on the container, for example an RFID card, which processing steps have to be carried out in the processing unit.

9. The device according to claim 8, **characterized in that** sensors are integrated into the container, which determine data about the container itself - such as the geographic position, the orientation, the temperature, etc. - and/or about the objects laid in or on said container - such as the temperature, the position inside the container, the orientation, etc. - and/or about its environment - such as the temperature, the air pressure, the time, etc. - and transmit them to the processing unit, so that these data may influence the processing.

10. The device according to any of claims 8 to 9, **characterized in that** reading and/or processing a start as soon as the container has been closed and the/or as soon as at least one predefined object and/or as soon as a predefined number of objects have been laid in or on the container.

11. The device according to any of claims 8 to 10, **characterized in that** among the objects storing data, appear objects which refer back to other objects which have not been laid in or on the container, and **in that** during the processing, a second reading unit also allows data to be read of those instruments back to which these objects refer.

12. The device according to any of claims 8 to 11, **characterized in that** at least one of the objects laid in or on the container is a device with a computing unit, such as a mobile phone, a smartphone, a portable minicomputer, a computer, a portable computer, a PDA, a watch and/or a tablet, and at least one of the objects laid in or on the container carries out at least portions of the processing.

13. The device according to any of claims 8 to 12, **characterized in that** the processing unit contains a transmission unit, which transmits data to another device or to several other devices, and/or a viewing unit which may be present on the actual container and/or on another device, and/or a statistical preparation unit and/or a backup unit and/or a reading unit which may modify or suppress data of the objects located in or on the container, different processing parameters being able to be extracted from the data of the objects are laid in the or on the container, such as the backup storage location, the size of the characters for viewing or the target device or the target address for transmitting the data.

14. The device according to any of claims 8 to 13, **characterized in that**, among the data, appear data for identifying persons and **in that** the data processing mode depends on the combination of the persons identified by means of the data, for example resulting from the use of an identification card of a patient and of an identification card of a physician, data of the patient being sent to the physician or from the use of several patient identification cards, statistical comparison of the data of all the identified patients being carried out.

## Revendications

1. Procédé pour l'enregistrement et le traitement de données, lesdites données étant stockées sur différents objets (11, 12, 13, 14, 15, 16, 18), tels que des appareils de mesure ou des cartes RFID, qui possèdent une interface pour la lecture des données, par exemple via NFC, RDID, Bluetooth, WLAN, ZigBee, ANT, IrDA Zeroconf, WPan, Z-Wave, Bonjour ou EnOcean, l'appareil de lecture (19) utilisé étant un contenant (17), tel qu'une valise ou une surface de pose spéciale ou un tapis de détection, dans ou sur lequel peuvent être posés tous les objets stockant les données, et les données des objets, qui ont été posés dans ou sur le contenant, pouvant être lues, **caractérisé en ce que** les données déterminent, sur au moins un (18) des objets posés dans ou sur le contenant, par exemple une carte RFID, quelles étapes de traitement doivent être mises en oeuvre.

2. Procédé selon la revendication 1, **caractérisé en ce que** le contenant lui-même peut déterminer avec des capteurs intégrés également des données sur lui-même - telles que la position géographique, l'orientation, la température, etc. - et/ou sur les objets posés dans ou sur ledit contenant - telles que la température, la position à l'intérieur du contenant, l'orientation, etc. - et/ou sur son environnement - telles que la température, la pression de l'air, l'heure, etc. - et ces données peuvent influencer le traitement.

3. Procédé selon l'une quelconque des revendications 1 à 2, **caractérisé en ce que** la lecture et/ou le traitement commencent dès que le contenant a été fermé et/ou dès qu'au moins un objet prédéfini et/ou dès qu'un nombre prédéfini d'objets ont été posés dans ou sur le contenant.

4. Procédé selon l'une quelconque des revendications 1 à 3 **caractérisé en ce que** parmi les objets stockant les données figurent des objets qui renvoient à d'autres objets qui n'ont pas été posés dans ou sur le contenant, et **en ce qu'**au cours du traitement sont également lues des données des appareils auxquels renvoient ces objets.

5. Procédé selon l'une quelconque des revendications 1 à 4, **caractérisé en ce qu'**au moins un des objets posés dans ou sur le contenant est un appareil avec une unité de calcul, tel qu'un téléphone mobile, un smartphone, un mini-ordinateur portable, un ordinateur, un ordinateur portable, un PDA, une montre et/ou une tablette, et au moins un des objets posés dans ou sur le contenant effectue au moins des parties du traitement.

6. Procédé selon l'une quelconque des revendications 1 à 5, **caractérisé en ce qu'**au cours du traitement est effectuée une transmission des données vers un autre appareil ou vers plusieurs autres appareils, et/ou une visualisation sur le contenant lui-même et/ou sur un autre appareil, et/ou une préparation statistique et/ou une sauvegarde et/ou une suppression ou un écrasement des données, différents paramètres de traitement pouvant être extraits des données des objets posés dans ou sur le contenant, tels que le lieu de stockage pour la sauvegarde, la taille des caractères pour la visualisation ou l'appareil cible ou l'adresse cible pour la transmission des données.

7. Procédé selon l'une quelconque des revendications 1 à 6, **caractérisé en ce que** parmi les données figurent des données d'identification de personnes et **en ce que** le mode de traitement des données dépend de la combinaison des personnes identifiées au moyen des données, du fait que, par exemple, grâce à l'utilisation d'une carte d'identification d'un patient et d'une carte d'identification d'un médecin, des données du patient sont envoyées au médecin, ou grâce à l'utilisation de plusieurs cartes d'identification de patients, une comparaison statistique des données de tous les patients identifiés est effectuée.

8. Dispositif pour l'enregistrement et le traitement de données, en particulier pour la mise en oeuvre du procédé selon l'une des revendications 1 à 7, constitué de différents objets (11, 12, 13, 14, 15, 16, 18) stockant des données, tels que des appareils de mesure ou des cartes RFID, qui possèdent une interface pour la lecture des données, par exemple via NFC, RDID, Bluetooth, WLAN, ZigBee, ANT, IrDA Zeroconf, WPan, Z-Wave, Bonjour ou EnOcean, et d'un contenant (17), tel qu'une valise, une boîte, une surface de pose spéciale ou un tapis de détection, qui possède une unité de lecture qui lit exclusivement des donnée des objets qui sont posés dans ou sur le contenant, et ledit dispositif contenant une unité de traitement, **caractérisé en ce que** les données déterminent, sur au moins un des objets posés dans ou sur le contenant, par exemple une carte RFID, quelles étapes de traitement doivent être mises en oeuvre dans l'unité de traitement.

9. Dispositif selon la revendication 8, **caractérisé en ce que** des capteurs sont intégrés dans le contenant, lesquels déterminent des données sur le contenant lui-même - telles que la position géographique, l'orientation, la température, etc. - et/ou sur les objets posés dans ou sur ledit contenant - telles que la température, la position à l'intérieur du contenant, l'orientation, etc. - et/ou sur son environnement - telles que la température, la pression de l'air, l'heure, etc. - et les transmettent à l'unité de traitement, de telle sorte que ces données peuvent influencer le traitement.

10. Dispositif selon l'une quelconque des revendications 8 à 9, **caractérisé en ce que** la lecture et/ou le traitement démarrent dès que le contenant a été fermé et/ou dès qu'au moins un objet prédéfini et/ou dès qu'un nombre prédéfini d'objets ont été posés dans ou sur le contenant.

11. Dispositif selon l'une quelconque des revendications 8 à 10, **caractérisé en ce que** parmi les objets stockant les données figurent des objets qui renvoient à d'autres objets qui n'ont pas été posés dans ou sur le contenant, et **en ce qu'**au cours du traitement, une deuxième unité de lecture permet également de lire des données des appareils auxquels renvoient ces objets.

12. Dispositif selon l'une quelconque des revendications 8 à 11, **caractérisé en ce qu'**au moins un des objets posés dans ou sur le contenant est un appareil avec une unité de calcul, tel qu'un téléphone mobile, un smartphone, un mini-ordinateur portable, un ordinateur, un ordinateur portable, un PDA, une montre et/ou une tablette, et au moins un des objets posés dans ou sur le contenant effectue au moins des parties du traitement.

13. Dispositif selon l'une quelconque des revendications 8 à 12, **caractérisé en ce que** l'unité de traitement contient une unité de transmission, qui transmet des données vers un autre appareil ou vers plusieurs autres appareils, et/ou une unité de visualisation qui peut être présente sur le contenant lui-même et/ou sur un autre appareil, et/ou une unité de préparation statistique et/ou une unité de sauvegarde et/ou une unité d'écriture qui peut modifier ou supprimer les données des objets situés dans ou sur le contenant, différents paramètres de traitement pouvant être extraits des données des objets posés dans ou sur le contenant, tels que le lieu de stockage pour la sauvegarde, la taille des caractères pour la visualisation ou l'appareil cible ou l'adresse cible pour la transmission des données.

14. Dispositif selon l'une quelconque des revendications 8 à 13, **caractérisé en ce que** parmi les données figurent des données d'identification de personnes et **en ce que** le mode de traitement des données dépend de la combinaison des personnes identifiées au moyen des données, du fait que, par exemple, grâce à l'utilisation d'une carte d'identification d'un patient et d'une carte d'identification d'un médecin, des données du patient sont envoyées au médecin ou grâce à l'utilisation de plusieurs cartes d'identification de patients, une comparaison statistique des données de tous les patients identifiés est effectuée.
